# EUROPEAN PATENT APPLICATION

(11) **EP 4 506 460 A1**
(43) Date of publication of application: **12.02.2025**
(21) Application number: 23784988.0
(22) Date of filing: 05.04.2023
(51) Int. Cl.: C12N 15/87, C12N 15/86, C12N 5/0783, A61K 35/17, A61P 35/00, A61P 31/00

(54) **METHOD FOR INTRODUCING TARGET GENE INTO IMMUNE CELLS**

(30) Priority: 06.04.2022 KR 20220042784
(71) Applicant: GC Cell Corporation, Yongin-si, Gyeonggi-do 16924 (KR)
(72) Inventor: KIM, Hansol, Yongin-si, Gyeonggi-do 16924 (KR); KIM, Hyun Ah, Yongin-si, Gyeonggi-do 16924 (KR); CHOI, Eunji, Yongin-si, Gyeonggi-do 16924 (KR); KIM, Yusun, Yongin-si, Gyeonggi-do 16924 (KR); KIM, Eunji, Yongin-si, Gyeonggi-do 16924 (KR); KIM, Hyojin, Yongin-si, Gyeonggi-do 16924 (KR); NAM, Hyeongjin, Yongin-si, Gyeonggi-do 16924 (KR); HAN, Seungryel, Yongin-si, Gyeonggi-do 16924 (KR); MIN, Bokyung, Yongin-si, Gyeonggi-do 16924 (KR); LIM, Hoyong, Yongin-si, Gyeonggi-do 16924 (KR); HWANG, Yu Kyeong, Yongin-si, Gyeonggi-do 16924 (KR)
(74) Representative: Fish & Richardson P.C.
(86) International application number: PCT/KR2023/004577
(87) International publication number: WO 2023/195764

(57) **Abstract**

The present invention relates to a method for efficiently expressing a target gene using a viral vector in immune cells, more specifically natural killer cells. By adding a simple process of freezing and thawing natural killer cells prior to transduction, the transduction efficiency of viral vectors can be dramatically improved without addition of enzymes or small molecules. The present invention is particularly useful for improving the efficiency of lentiviral vector that has a low gene transfer efficiency into natural killer cells, thereby obtaining natural killer cells in which desired genes are introduced to maximize their function, activity, and productivity as a source of immunotherapy.

## Description

### Technical Field

The present invention relates to a method for improving the transduction efficiency of a target gene in immune cells, specifically natural killer cells, via a viral vector by freezing and thawing the cells.

### Background Art

Natural killer (NK) cells are lymphocytes that account for about 15% of blood cells and play an important role in the innate immune defense against malignant cells. Specifically, natural killer cells activate dendritic cells and induce cytotoxic T lymphocytes (CTLs) to respond specifically to tumors to eliminate tumor cells, most notably by directly killing malignant tumors such as sarcoma, myeloma, carcinoma, lymphoma, and leukemia. However, immune cell therapy using natural killer cells has been limited by difficulties in *ex vivo* expanding and differences in natural killer cell activity between patients.

Meanwhile, in immune cell therapy with T cells, methods are being developed to reprogram autoimmune cells to express chimeric antigen receptors to increase effectiveness against specific cancers or to overcome the resistance of cancer cells to treatment. These chimeric antigen receptors have recently been applied to natural killer cells. In addition, in treatments using allogeneic natural killer cells, there is a need to introduce foreign genes or suppress or overexpress intrinsic genes to maximize their function and activity as immunotherapeutic cells, such as expressing the cytokine IL-2 to induce NK cell proliferation thereby overcome the short persist of transplanted NK cells in the recipient; or suppressing inhibitory receptors (such as KIRs) on NK cells to block the recipient's immune response. However, natural killer cells have the disadvantage of low transduction efficiency, especially using viral vectors such as lentiviruses. Therefore, there has been a continuous need to develop more efficient gene transduction methods.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop a method for efficiently expressing various target genes in immune cells to maximize the function, activity, and productivity of immune cells as a therapeutic cell resource. As a result, the present inventors have discovered that freezing and thawing immune cells, specifically natural killer cells, prior to introducing target genes using viral vectors significantly improves the efficiency of gene delivery and enables effective acquisition of natural killer cells with phenotypes optimized for immunotherapy, thereby completing the present invention.

Accordingly, it is an object of this invention to provide a method for expressing a target gene in immune cells.

It is another object of this invention to provide a pharmaceutical composition for the preventing or treating a tumor or infectious disease comprising a natural killer cell expressing a target gene by the method of the present invention.

Other objects and advantages of the present invention will become more apparent from the following detailed description of the invention, the appended claims and the accompanying drawings.

### Technical Solution

In one aspect of this invention, there is provided a method for expressing a target gene in an immune cell comprising:
(a) Freezing the immune cell;
(b) thawing the frozen immune cell; and
(c) introducing a gene delivery system comprising the target gene into the thawed immune cells.

The present inventors have made intensive studies to develop a method for efficiently expressing various target genes in immune cells to maximize the function, activity, and productivity of immune cells as a therapeutic cell resource. As a result, the present inventors have discovered that freezing and thawing immune cells, specifically natural killer cells, prior to introducing target genes using viral vectors significantly improves the efficiency of gene delivery and enables effective acquisition of natural killer cells with phenotypes optimized for immunotherapy.

As used herein, the term "target gene" refers to a nucleic acid molecule that is inserted into a gene delivery system to be artificially expressed in an immune cell or to be artificially increased its natural expression, to gain a desired trait in the target immune cell. As used herein, the term "nucleic acid molecule" has a meaning comprehensively including DNA (gDNA and cDNA) and RNA molecules, and nucleotides, which are the basic structural units in nucleic acid molecules, include not only natural nucleotides, but also analogues in which sugar or base sites are modified (Scheit, Nucleotide Analogs, John Wiley, New York(1980); Uhlman and Peyman, Chemical Reviews, 90:543-584(1990)).

As used herein, the term "express" refers to causing a subject cell to express an exogenous gene or to increase the natural expression of an endogenous gene by artificially introducing the gene using a gene delivery system so that the gene becomes replicable in the subject cell as an extrachromosomal factor or by completion of chromosomal integration. Thus, the term "expression" has the same meaning as "transformation", "transduction" or "transduction".

As used herein, the term "gene delivery system" refers to any means for delivering a gene into a cell, and gene delivery has the same meaning as intracellular transduction of a gene. At the tissue level, the term "gene transfer" has the same meaning as the spread of a gene. Therefore, the gene delivery system of the present invention may be described as a gene transduction system and a gene spreading system.

In order to produce the gene delivery system of the present invention, the nucleotide sequence of the present invention is preferably present in a suitable expression construct. In the expression construct, the nucleotide sequence of the present invention is preferably operatively linked to a promoter. As used herein, the term "operatively linked" refers to a functional linkage between a nucleic acid expression regulatory sequence (e.g., a promoter, a signal sequence, or an array of transcription regulation factor binding sites) and another nucleic acid sequence, and through the linkage, the regulatory sequence regulates the transcription and/or translation of the other nucleic acid sequence. The promoter linked to the nucleotide sequence of the present invention is one that can regulate the transcription of the target gene by action specifically in animal cells, more specifically mammalian cells, includes, for example, promoters derived from mammalian viruses and promoters derived from mammalian cell genomes. Examples of the promoter include, but are not limited to, mammalian cytomegalovirus (CMV) promoter, adenovirus late promoter, vaccinia virus 7.5K promoter, SV40 promoter, HSV *tk* promoter, RSV promoter, EF1 alpha promoter, metallothionein promoter, beta-actin promoter, human IL-2 gene promoter, human IFN gene promoter, human IL-4 gene promoter, human lymphotoxin gene promoter, and human GM-CSF gene promoter.

The nucleotide sequence of the target gene may be applied to any gene delivery system used for ordinary gene introduction. Preferably, the nucleotide sequence of the target gene may be applied to plasmids, adenoviruses (Lockett LJ, et al., Clin. Cancer Res. 3:2075-2080(1997)), adeno-associated viruses (AAV) (Lashford LS., et al., Gene Therapy Technologies, Applications and Regulations Ed. A. Meager, 1999), retroviruses (Gunzburg WH, et al., Retroviral vectors. Gene Therapy Technologies, Applications and Regulations Ed. A. Meager, 1999), lentiviruses (Wang G. et al., J. Clin. Invest. 104(11):R55-62(1999)), herpes simplex virus (Chamber R., et al., Proc. Natl. Act . Sci USA 92:1411-1415(1995)), vaccinia viruses (Puhlmann M. et al., Human Gene Therapy 10:649-657(1999)), liposomes (Methods in Molecular Biology, 199, S.C. Basu and M. Basu (Eds.), Human Press 2002) or niosomes.

According to a specific embodiment, the gene delivery system utilized in the present invention is a viral vector.

More specifically, the virus is selected from the group consisting of lentiviruses, adenoviruses, adeno-associated viruses (AAVs), retroviruses, herpes simplex viruses and vaccinia viruses, and most specifically, lentiviruses.

In the present invention, when the gene delivery system is constructed based on a viral vector, the contacting step is performed according to a viral infection method known in the art. The infection of host cells using viral vectors are described in the above-mentioned cited documents.

As used herein, the term "immune cell" refers to any cell involved in the initiation or promotion of immune response, and more specifically refers to immune effector cells. Immune cells include, but are not limited to, for example, T cells, B cells, natural killer (NK) cells, natural killer T (NKT) cells, and mast cells. More specifically, the immune cells are natural killer cells.

As used herein, the term "freezing" or "cryopreservation" refers to stably maintaining cells for a long period of time in a low temperature state, more specifically, in an ultra-low temperature state of -70°C to -200°C. Generally, when cells are cultured, a mutation occurs in a ratio of one to ten thousand, and when cells go through a long-term subculture, cell populations may change and become different from the original populations. In severe cases, cells may lose their own particular functions by subculture. Further, cells may be infected with mycoplasma or the like during subculture. Because of such problems, cell cryopreservation is performed to freeze and preserve cells before losing their intrinsic characteristics, and to use them when needed. The present inventors have discovered for the first time that the efficiency of gene transfer in immune cells, especially in natural killer cells where the efficiency of gene introduction using lentiviral vectors is very low, is greatly improved in case such a freezing process is preceded by a gene transfer. Thus, the freezing step performed in the present invention may be performed only for a short period of time, in contrast to cryopreservation for the purpose of long-term preservation of cells.

Freezing of cells can be performed by treating a cryoprotectant that can minimize damage to the cells due to the formation of ice crystals and ionic and osmotic imbalances that inevitably accompany the freezing and thawing process. The freezing medium may include, for example, dextran, albumin and/or DMSO to improve the safety and stability of the cells during freezing.

As used herein, the term "thawing" refers to the process of raising the temperature of frozen or cryopreserved cells until they become soft, living cells in order to allow them to resume vital activity. Thawing can typically be accomplished quickly by removing cryogenically frozen cells from a liquid nitrogen tank and placing them in a 37°C constant temperature water bath.

Since the freezing and thawing process of the present invention is aimed at improving the efficiency of gene transduction into cells rather than long-term storage of cells, the freezing (step (a) of the present invention) and thawing (step (b) of the present invention) may be performed without a temporal interval and the thawing process may be initiated immediately after the freezing is completed, or a suitable time interval may be left between the freezing and thawing processes as needed.

According to a specific embodiment, the method of the invention further comprises culturing the immune cell isolated from the subject *in vitro* for 6 to 11 days prior to the step (a). More specifically, the method further comprises culturing for 6 to 9 days, more specifically for 6 to 8 days, most specifically for about 7 days prior to the step (a).

According to a specific embodiment, the method of the invention further comprises culturing the thawed immune cell for 2 to 4 days after the step (b). More specifically, the method further comprises culturing for 3 to 4 days, more specifically for 2 to 3 days, most specifically for about 3 days after the step (b).

According to a specific embodiment, the step (c) is performed by adding TAK1 MAPKKK (transforming growth factor-β-activated kinase 1 mitogen-activated protein kinase kinase kinase kinase) inhibitor, a TBK1 (TANK Binding Kinase 1) inhibitor, or a combination thereof to the culture medium of the thawed immune cells.

Specifically, the TAK1 MAPKKKK inhibitor may be a compound represented by Formula 1 below, or a pharmaceutically acceptable salt thereof:

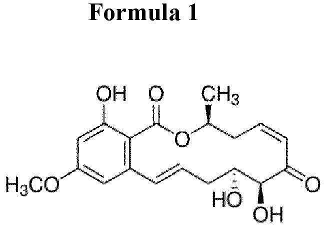

Specifically, the TBK1 inhibitor may be a compound represented by Formula 2 below, or a pharmaceutically acceptable salt thereof:

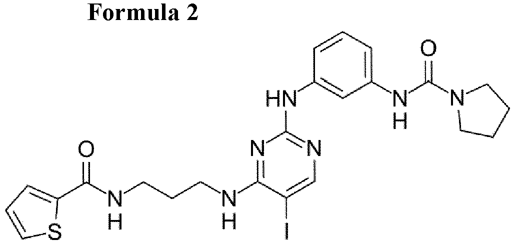

As used herein, the term "pharmaceutically acceptable salt" includes a salt derived from a pharmaceutically acceptable inorganic acid, organic acid, or base. Examples of suitable acids include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluoroacetic acid, citric acid, methanesulfonic acid, formic acid, benzoic acid, malonic acid, naphthalene-2-sulfonic acid, benzenesulfonic acid, and the like. Salts derived from suitable bases can include alkali metals such as sodium, alkaline earth metals such as magnesium, and ammonium.

In another aspect of this invention, there is provided a natural killer cell expressing a target gene by the method of the present invention described above.

The methods of introducing target genes and the immune cells (e.g., natural killer cells) into which the target genes are introduced have already been described in detail, and are therefore omitted to avoid undue redundancy.

In still another aspect of this invention, there is provided a pharmaceutical composition for preventing or treating a tumor or infectious disease comprising the natural killer cell of the present invention as an active ingredient.

In still another aspect of this invention, there is provided a method for preventing or treating a tumor or infectious disease comprising administering to a subject in need thereof a natural killer cell of the present invention.

When the present invention is applied to immune cells, it may be applied for the treatment of tumors and infectious diseases. The NK cells produced by the method according to the present invention may be applied to all types of tumors, including solid cancer and blood cancer. Unlike blood cancer, solid cancer refers to cancer formed as a lump in an organ, and includes cancers occurring in most organs. Tumors that may be treated using the NK cells according to the present invention are not specially limited and include gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, lymphoma, etc., without being limited thereto. Infectious diseases that can be treated using the immune cells of the present invention are diseases caused by infection with viruses or pathogens, and include all diseases that may be caused by infection through respiratory organs, blood, skin contact, etc. These infectious diseases include, but are not limited to, for example, hepatitis B and C, human papilloma virus (HPV) infection, cytomegalovirus infection, viral respiratory diseases, and influenza.

As used herein, the term "treating" refers to (a) inhibiting the development of a disease, disorder or symptom; (b) alleviating the disease, disorder or symptom; or (c) eliminating the disease, disorder or symptom. When the stem cells into which the immunogenicity inhibitory composition of the present invention has been introduced are administered to a subject, they serves to inhibit the development of symptoms caused by excessive or unwanted immune responses, or eliminate, or alleviate the symptoms, and when the immune cells into which the immunogenicity inhibitory composition of the present invention has been introduced are administered to a subject, they serve to induce the death of cancer cells or infected cells, thereby inhibiting the development of symptoms caused by tumors or infectious diseases, or eliminating or alleviating the symptoms. Accordingly, the composition of the present invention may be a cell therapeutic composition for a disease by itself, or may be administered together with other pharmacological components and applied as a therapeutic adjuvant for the disease. Accordingly, as used herein, the term "treatment" or "therapeutic agent" includes the meaning of "therapeutic adjuvant" or "therapeutic adjuvant".

As used herein, the term "administration" or "administering" refers to directly administering a therapeutically effective amount of the composition of the present invention to a subject so that the same amount is formed in the subject's body.

As used herein, the term "therapeutically effective amount" refers to an amount of the composition that is sufficient to provide a therapeutic or prophylactic effect to a subject to which the composition of the present invention is to be administered, and thus the term is meant to include a "prophylactically effective amount".

As used herein, the term "subject" includes, without limitation, a human, mouse, rat, guinea pig, dog, cat, horse, cow, pig, monkey, chimpanzee, baboon or rhesus monkey. Specifically, the subject of the present invention is a human.

**In** still another aspect of this invention, there is provided a method for increasing the efficiency of a target gene expression in an immune cell, comprising freezing the immune cell; and thawing the frozen immune cell.

The immune cells used in the present invention, the freezing and thawing process thereof, and the method for delivering the target gene have already been described in detail, and are therefore omitted to avoid undue redundancy.

### Advantageous Effects

The features and advantages of the present invention are summarized as follows:
(a) The present invention provides a method for efficiently expressing a target gene using a viral vector in immune cells, more specifically natural killer cells
(b) By adding a simple process of freezing and thawing natural killer cells prior to transduction, the method of the present invention dramatically improves the transduction efficiency of viral vectors without addition of enzymes or small molecules.
(c) The present invention is particularly useful for improving the efficiency of lentiviral vector that has a low gene transfer efficiency into natural killer cells, thereby obtaining natural killer cells in which desired genes are introduced to maximize their function, activity, and productivity as a source of immunotherapy.

### Brief Description of Drawings

FIG. 1 shows the expression efficiency of HER2 CAR introduced by lentiviral vectors after freezing cells at various time points during NK cell culture and incubation for 3 days from the time of freezing.
FIG. 2 shows the efficiency of HER2 CAR gene transduction using lentiviral vectors in NK cells cultured for 3 days after freezing at various time points and in culture without freezing.
FIG. 3 shows the comparison of transduction efficiency of lentiviral vector according to post-thaw culture period of frozen NK cells.
FIG. 4 shows the comparison of transduction efficiency of lentiviral vector according to post-thaw culture period (FIG. 4a) and the comparison of efficiency with and without freezing of NKs on day 10 of culture (FIG. 4b), as confirmed by FACS analysis.
FIG. 5 shows the efficiency of lentiviral vector delivery into NK cells using the enhancers BX795 (FIG. 5a) and 5z-7-oxozeaenol (FIG. 5b).
FIG. 6 shows the transduction efficiency of lentiviral vectors expressing various CAR scFvs as determined by the CAR expression rate (FIG. 6a) and Vector Copy Number (VCN) values (FIG. 6b), respectively.

### Mode for Invention

Hereinafter, the present invention will be described in more detail with reference to examples. These examples are only for illustrating the present invention in more detail, and it will be apparent to those of ordinary skill in the art that the scope of the present invention according to the subject matter of the present invention is not limited by these examples.

### Examples

### Example 1: Comparison of lentiviral vector transduction efficiency of NK cells at different freezing times

### 1-1) NK cell culture and freezing

Cord blood natural killer (CBNK) cells were isolated and cultured from human donors. The cells were stimulated with feeder cells on first day of culture and cultured using CD4+ T cells transduced with 4-1BBL, membrane bound IL-21 (mbIL-21) and mTNF-a (membrane TNF-α) genes as feeder cells. CellGro^{®} serum-free medium (Cellgenix) containing 1% (v/v) human plasma, 1333 IU hIL-2 (Proleukin, Novartis, Korea), and 4 mM glutamine (Hyclone) was used as NK cell culture medium, and the cell concentration was adjusted to 1 x 10⁶ /mL for NK cell culture and cultured in appropriate volume culture vessels.

As the cryopreservation medium, PBS (Irvine Scientific) containing 25% dextran-40 (Hospira), 20% albumin (Akron Biotech), and 5% DMSO (Avantor) was used. NK cells were frozen using a freezing container or an automated cell freezer, and then stored in a -70°C ultra-low temperature freezer or in an ultra-low temperature liquid nitrogen tank below -130°C until use.

### 1-2) NK cell transduction with lentiviral vectors

The conditions for cell freezing, transduction (TD), and expression checks at each time point of NK culture are shown in Table 1 below. NK cells were cultured for 3, 5, 7, 9, 11, and 13 days from day 0 (immediately after isolation), frozen, and thawed simultaneously after being stored at ultra-low temperatures. NK cells were transfected with anti-HER2 scFv CAR (HER2 CAR) gene by coculture with NK culture medium for 3 days as a recovery period to prevent possible cell damage during the freezing and thawing process. After thawing and culturing for 3 days, NK cells were counted by hemocytometer and 3 x 10⁵ NK cells were seeded into each well of a 48-well plate. Lentiviral vectors to express the HER2 CAR gene were produced by ICGM (Daejeon, Korea). To transfect HER2 CAR into NK cells, a 20 MOI (multiplicity of infection) complex of HER2 CAR lentiviral vector and 10 µg/mL Protransduzin (JPT, TE-PTDB-2) was prepared and left at room temperature for at least 5 min before treatment of NK cells in 48-well plates. The culture medium was supplemented with 20 ng/mL IL-21 (Biolegend/571204) to promote the intracellular introduction and expression of the lentiviral vector. The day after transduction, fresh NK cell culture medium containing 20 ng/mL of IL-21 was added in equal volumes to each well of the plate.

**[Table 1] Time point of freeze, transduce, and check the expression during NK cells culture**

| **NK cell** | **Freezing** | **Transduction** | **Confirmation of CAR expression (transduction + 6 days)** |
|---|---|---|---|
| D0F-TD3 | Day 0 | Day 3 | Day 9 |
| D3F-TD6 | Day 3 | Day 6 | Day 12 |
| D5F-TD8 | Day 5 | Day 8 | Day 14 |
| D7F-TD10 | Day 7 | Day 10 | Day 16 |
| D9F-TD12 | Day 9 | Day 12 | Day 18 |
| D11F-TD14 | Day 11 | Day 14 | Day 20 |
| D13F-TD16 | Day 13 | Day 16 | Day 22 |

### 1-3) Confirmation of expression of the transfected gene in NK cells

HER2 CAR expression was determined by flow cytometry (FACS) in NK cells cultured for 6 days after transduction. NK cells thawed and cultured for 9 days without HER2 CAR transduction were used as HER2 CAR negative controls. 2 X 10⁵ NK cells were collected, centrifuged at 1,200 rpm for 5 minutes, supernatant removed, and resuspended in 2 mL of FACS buffer supplemented with 2% FBS. The supernatant was removed by centrifugation at 2000 rpm for 3 minutes and resuspended in 100 µL FACS buffer with 1 µL of recombinant human HER2 protein (R&D systems) conjugated to 6 X histidine at the C-terminus at 50 ng/µL and reacted for 30 minutes at 4°C. Tubes for subtype staining were reacted under the same conditions with 100 µL of FACS buffer without recombinant human HER2 protein. The tubes were then centrifuged at 2000 rpm for 3 minutes with 2 mL of FACS buffer and the supernatant was removed. For the secondary staining, the washed NK cells were loaded with 100 µL FACS buffer, followed by the antibodies to be assayed as shown in Table 2 and reacted at 4°C for 30 minutes. The cells were then washed with 2 mL of FACS buffer, fixed with 200 µL fixation solution (BD, 554655), and analyzed for expression on the NK cell surface by flow cytometry FACS (LSRFortessa, BD Bioscience).

**[Table 2] Antibody for FACS analysis to confirm anti-HER2 scFv CAR expression on NK cells**

| **Markers** | **Antibody (fluorescence/manufacturer/ product number)** | **Subtypes** | **HER2 CAR** |
|---|---|---|---|
| Anti-human CD56 | PEcy7 / BD Bioscience / 557747 | 1 µL | 1 µL |
| Anti-human CD3 | APC / BD Bioscience / 555335 | 4 µL | 4 µL |
| 7-AAD | PerCp-Cy5.5/ Beckmen Coulter/ A07704 | 4 µL | 4 µL |
| Anti-6x histidine | PE /Abcam /Ab72467 | 1 µL | 1 µL |

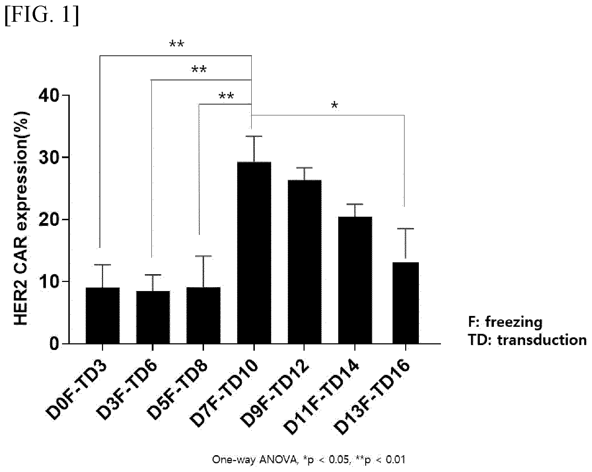

Anti-HER2 scFv CAR gene transduction efficiency was compared among NK cells with 7-AAD-negative, CD3-negative, and CD56-positive phenotypes by calculating the % of cells with positive HER2 CAR expression relative to the subtype-stained cells. When NK cells frozen on day 0, day 3, and day 5 were thawed and cultured for 3 days before transduction with lentiviral vectors on day 3 (D0F-TD3), day 6 (D3F-TD6), and day 8 (D5F-TD8), respectively, HER2 CAR expression was low, ranging from 8-9%. However, in NK cells transduced on day 10 (D7F-TD10) by thawing frozen NK cells on day 7 and culturing them for 3 days, HER2 CAR expression increased rapidly to 29.3%. In NK cells thawed after being frozen on days 9, 11, and 13 of culture in the same manner and then cultured for 3 days, showed a decrease over time, with 26.4%, 20.5%, and 13.7% expression on days 11 (D9F-TD11), 13 (D11F-TD13), and 15 (D13F-TD15) of culture, respectively (FIG. 1). The detailed values of the graph in Figure 1 are summarized in Table 3. As shown in Figure 1, when cells were frozen, thawed and transduced with lentiviral vectors at various time points during NK cell culture, gene expression efficiency was higher when cells were frozen and transduced after day 7 than at the beginning of culture, with the highest expression in the D7F-TD10 condition, where cells were frozen and thawed at day 7 and transduced on day 10 after 3 additional days of culture.

**[Table 3] HER2 CAR expression % according to time point of freeze and TD**

| Freeze-TD time | HER2 CAR % (standard deviation) |
|---|---|
| D0F-TD3 | 9.1 (±3.6) |
| D3F-TD6 | 8.5 (±2.6) |
| D5F-TD8 | 9.1 (±5.0) |
| D7F-TD10 | 29.3 (±4.1) |
| D9F-TD12 | 26.4 (±2.0) |
| D11F-TD14 | 20.5 (±2.0) |
| D13F-TD16 | 13.7 (±4.8) |

### Example 2: Comparison of gene transduction efficiency with and without freezing NK cells

Table 4 shows the detailed culture schedule and experimental conditions to compare the efficiency of gene transduction with and without freezing NK cells. HER2 CAR expression results were compared between the frozen NK cells cultured for 3 days after thawing and gene transduction (Freeze-thaw) and the NK cells in which the gene was transduced after the same culture period without freezing (Fresh).

**[Table 4] Time point of freeze, transduce and check expression of NK cells in culture**

| **NK cells** | **Freezing** | **Transduction** | **Confirmation of CAR expression (transduction +6 days)** |
|---|---|---|---|
| **TD6** | Not applicable | Day 6 | Day 12 |
| **D3F-TD6** | Day 3 | Day 6 (Thaw + 3 days) | Day 12 |
| **TD8** | Not applicable | Day 8 | Day 14 |
| **D5F-TD8** | Day 5 | Day 8 (Thaw + 3 days) | Day 14 |
| **TD10** | Not applicable | Day 10 | Day 16 |
| **D7F-TD10** | Day 7 | Day 10 (Thaw + 3 days) | Day 16 |
| **TD12** | Not applicable | Day 12 | Day 18 |
| **D9F-TD12** | Day 9 | Day 12 (Thaw + 3 days) | Day 18 |
| **TD14** | Not applicable | Day 14 | Day 20 |
| **D11F-TD14** | Day 11 | Day 14 (Thaw + 3 days) | Day 20 |
| **TD16** | Not applicable | Day 16 | Day 22 |
| **D13F-TD16** | Day 13 | Day 16 (Thaw + 3 days) | Day 22 |

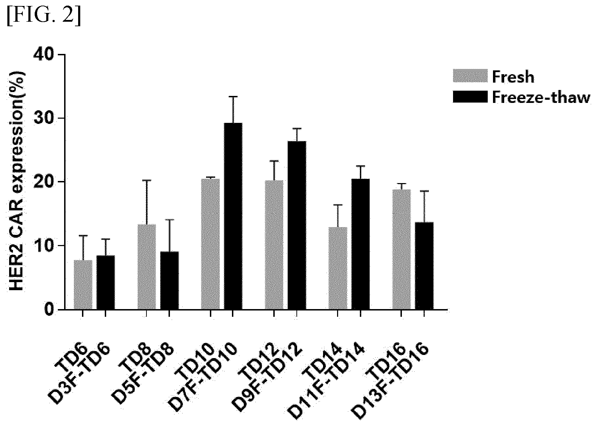

The experimental group that underwent the freezing and thawing process was subjected to cell culture and lentiviral vector transduction in the same way as in Example 1. NK cells in the non-freezing group were cultured without the freezing process after feeder cell stimulation on day 0 of culture and transduced with lentiviral vectors on days 6, 8, 10, 12, 14, and 16 of culture, respectively. Gene transduction was performed in the same manner as in Examples 1-2. All experimental groups were cultured for 6 days after gene transduction, and then the expression of the transgenes was analyzed by FACS the same way as in Examples 1-3. As a result, the highest HER2 CAR expression efficiency was found in D7F-TD10, where the frozen NK cells were thawed on day 7 of culture and then transfected on day 10 of culture (FIG. 2). The detailed values of each graph in Figure 2 are shown in Table 5. Furthermore, when comparing days 10 and 14 of culture, the efficiency of HER2 CAR expression was higher in NK cells transfected after freezing than in NK cells transfected without freezing. From the above results, it can be concluded that transduction with lentiviral vectors after the same culture period has a higher gene transduction efficiency in NK cells that have undergone the freeze-thaw process, and that the freeze on day 7/ transduction on day 10 of culture (D7F-TD10) is the optimal condition for introducing foreign genes into NK cells (FIG. 2, Table 5).

**[Table 5]**

| **NK Cells (Fresh)** | | **NK cells (Freeze-thaw)** | |
|---|---|---|---|
| **TD point in time** | **HER2 CAR % (standard deviation)** | **When to freeze-TD** | **HER2 CAR % (standard deviation)** |
| TD6 | 7.8 (±3.8) | D3F-TD6 | 8.6 (±2.6) |
| TD8 | 13.4 (±6.8) | D5F-TD8 | 9.1 (±5.0) |
| TD10 | 20.5 (±0.3) | D7F-TD10 | 29.3 (±4.1) |
| TD12 | 20.2 (±3.1) | D9F-TD12 | 26.4 (±2.0) |
| TD14 | 12.9 (±3.4) | D11F-TD14 | 20.5 (±2.0) |
| TD16 | 18.8 (±1.0) | D13F-TD16 | 13.7 (±4.8) |

### Example 3: Comparison for transduction efficiency of lentiviral vectors according to culture day after NK cell thawing

Since it was confirmed from the results of Examples 1 and 2 that freezing the NK cells at the 7th day of culture had the highest transduction efficiency, the transduction efficiency of lentiviral vectors was compared under the experimental conditions in Table 6 below according to the incubation period for cell recovery after thawing of NK cells frozen at the 7th day of culture.

**[Table 6] Time point of freeze, transduce, and check expression of NK cells in culture**

| **NK cells** | **Freezing** | **Transduction** | **Gene expression confirmation (transduction + 6 days)** |
|---|---|---|---|
| D7F-TD7 | Day 7 | Day 7 (just after thawing) | Day 13 |
| D7F-TD8 | | Day 8 (Thaw +1 day) | Day 14 |
| D7F-TD9 | | Day 9 (Thaw +2 days) | Day 15 |
| D7F-TD10 | | Day 10 (Thaw +3 days) | Day 16 |
| D7F-TD11 | | Day 11 (Thaw +4 days) | Day 17 |

Transduction of lentiviral vectors was performed immediately after thawing (D7F-TD7), on day 1 of post-thaw incubation (D7F-TD8), on day 2 of post-thaw incubation (D7F-TD9), on day 3 of post-thaw incubation (D7F-TD10), and on day 4 of post-thaw incubation (D7F-TD11), using the same experimental methods as in Examples 1-2, respectively. Four types of lentiviral vectors (Lentigen, MD, USA) expressing anti-HER2 scFv CAR (HER2 CAR), anti-CD5 scFv CAR (CD5 CAR), anti-CD19 scFv CAR (CD19 CAR) and GFP gene were used. The expression of HER2 CAR, CD5 CAR, and CD19 CAR in NK cells cultured for 6 days after transduction was measured in the same manner as in Examples 1-3. The recombinant proteins used to analyze the expression of each gene and the antibodies used for secondary staining are summarized in Table 7 and Table 8, respectively. After secondary staining, NK cells were fixed with 200 µL fixation solution, and the CAR gene transduction efficiency was compared by calculating the % value of cells positive for HER2 CAR or CD5 CAR or CD19 CAR expression relative to the subtype-stained cells among 7-AAD-negative, CD3-negative, and CD56-positive cells. GFP gene transduction efficiency was compared by calculating the mean FITC fluorescent intensity (MFI) value of 7-AAD-negative, CD3-negative, and CD56-positive cells relative to negative control NKs

**[Table 7] Time point of freeze, transduce, and check expression of NK cells in culture**

| **Introgenes** | **Markers** | **Recombinant proteins (tag/manufacturer/art icle number)** | **Subtypes** | **HER2, CD5 or CD19 CAR** |
|---|---|---|---|---|
| Anti-HER2 scFv CAR | Human HER2 protein (50 ng/uL) | 6x Histidine/ R&D systems/ 1129-ER | 0 µL | 1 µL |
| Anti-CD5 scFv CAR | Human CD5 protein (50 ng/uL) | Biotin/Sinobiological /11027-H27H-B | 0 µL | 1 µL |
| Anti-CD19 scFv CAR | Human CD19 protein (1 test/2uL) | Biotin/Acro/FM3-BY54 | 0 µL | 2 µL |
| | | | | |

**[Table 8] Antibodies for FACS analysis to confirm expression of transgenes in NK cells**

| **Introgenes** | **Markers** | **Antibody (fluorescence/ manufacturer/product number)** | **Subtypes** | **HER2, CD5, or CD19 CAR** |
|---|---|---|---|---|
| Common | Anti-human CD56 | PEcy7/ BD Bioscience/ 557747 | 1 µL | 1 µL |
| | Anti-human CD3 | APC/ BD Bioscience/ 555335 | 4 µL | 4 µL |
| | 7-AAD | PerCp-Cy5.5/ Beckmen Coulter/ A07704 | 4 µL | 4 µL |
| Anti-HER2 scFv CAR | Anti-6x histidine | PE / Abcam / Ab72467 | 1 µL | 1 µL |
| Anti-CD5 and anti-CD19 scFv CARs | Streptavidin | PE / BD bioscience / 554061 | 0.2 µL | 0.2 µL |

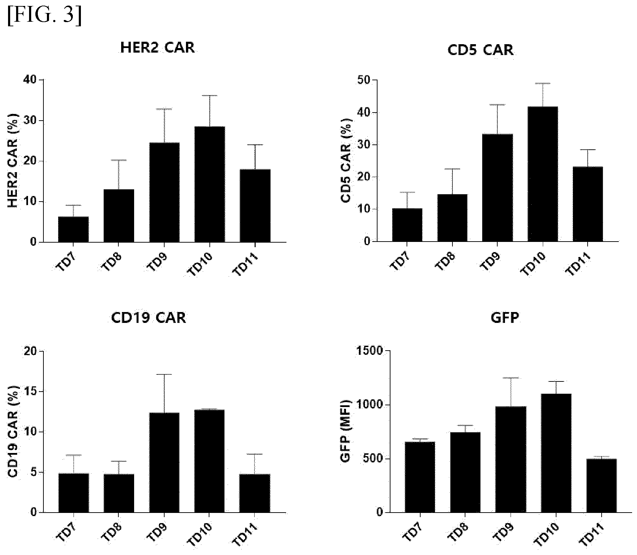

NK cells frozen at day 7 of culture showed gradually higher transgene expression in the day 1, day 2, and day 3 post-thaw conditions than immediately after thawing, resulting in the highest transduction efficiency at day 3 post-thaw, i.e., day 10 of NK culture, followed by a decrease in transduction efficiency from day 4 onward. All four different transfected genes showed similar gene expression trends, confirming that the D7F-TD10 condition, in which frozen NK cells were thawed on day 7 of culture and then transfected for 3 days, was the most efficient lentiviral vector transduction condition in NK cells (FIG. 3). The detailed values of each graph in Figure 3 are shown in Table 9.

**[Table 9] Transgene expression by incubation period after thawing**

| **After defrosting the D7F TD point in time** | **HER2 CAR (standard deviation)** | **CD5 CAR (standard deviation)** | **CD19 CAR (standard deviation)** | **GFP MFI (standard deviation)** |
|---|---|---|---|---|
| TD7 | 6.2 (±2.8) | 10.3 (±5.0) | 4.9 (±2.3) | 652.7 (±28.7) |
| TD8 | 13.0 (±7.2) | 14.7 (±7.9) | 4.8 (±1.6) | 743.0 (±64.2) |
| TD9 | 24.5 (±8.3) | 33.4 (±9.0) | 12.4 (±4.8) | 982.8 (±266.5) |
| TD10 | 28.5 (±7.6) | 41.9 (±7.2) | 12.7 (±0.1) | 1102.1 (±114.1) |
| TD11 | 18.0 (±6.1) | 23.3 (±5.3) | 4.8 (±2.5) | 499.6 (±22.6) |

### Example 4: Transduction of HER2 CAR into NK cells using the D7F-TD10 method

NK cells were cultured for 10 days without freezing process and the transduction efficiency at various time points was compared with the expression of HER2 CAR. For the 10-day culture condition, the transduction efficiency was compared with the D7F-TD10 condition in which NKs frozen on the 7th day of culture were thawed and cultured for 3 days, and the detailed comparison conditions are shown in Table 10.

**[Table 10] Time point of freeze, transduce, and check expression of NK cells in culture**

| **NK cells** | **Freezing** | **Transduction** | **Confirmation of CAR expression (+6 days after transduction)** |
|---|---|---|---|
| **Untransduced** | Not applicable | Not applicable | Incubation Day 16 |
| **TD3** | Not applicable | Day 3 | Day 9 |
| **TD7** | Not applicable | Day 7 | Day 13 |
| **TD10** | Not applicable | Day 10 | Day 16 |
| **D7F-TD10** | Day 7 | Day 10 (Thaw +3 days) | Day 16 |

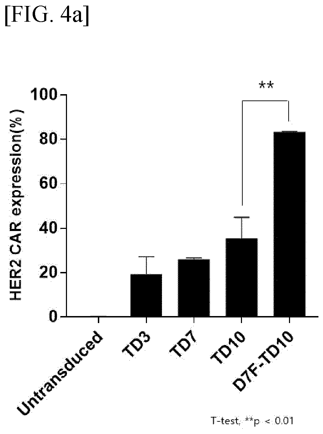

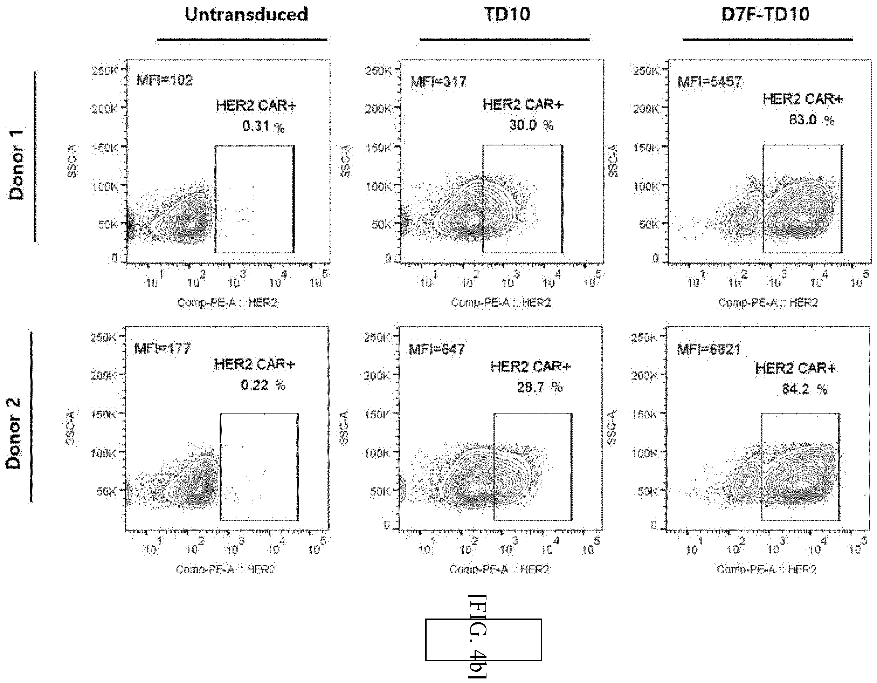

Umbilical cord blood-derived NK cells were cultured using feeder cells in the same manner as in Example 1-1, and HER2 CAR gene transduction was performed using lentiviral vectors on days 3, 7, and 10 of culture, respectively. NK cells in the D7F-TD10 condition were frozen on day 7 of culture and thawed and cultured for 3 days for HER2 CAR gene transduction. The detailed gene transduction method is the same as in Examples 1-2, where NK cells are treated with 20 MOI of lentiviral vector expressing HER2 CAR, 10 µg/mL protransduzin, and 20 ng/mL human IL-21 recombinant protein. In addition, NK cells were treated with (5Z)-7-oxozeaenol (TOCRIS, 3604), a transforming growth factor-β-activated kinase 1 (TAK1) inhibitor known as an enhancer to increase lentiviral transduction efficiency, at a concentration of 6 µM. The day after transduction, fresh NK cell culture medium containing 20 ng/mL of IL-21 and 6 µM (5Z)-7-oxozeaenol was added in equal amounts to each well of the plate. NK cells cultured for 6 days after transduction were checked for HER2 CAR expression as a % value analyzed by FACS in the same manner as in Examples 1-3. In the case of NK cells without freezing process, HER2 CAR expression increased with increasing culture period, with the highest HER2 CAR expression in TD10, but the gene transfuction efficiency of the D7F-TD10 condition was significantly higher even though the NK cells were cultured for the same period (FIG. 4a). Furthermore, FACS counter plot analysis comparing the expression of NK cells transfected with HER2 CAR in TD10 or D7F-TD10 conditions showed that D7F-TD10 had more than 2-fold higher HER2 CAR expression than TD10, and MFI was also more than 10-fold higher (FIG. 4b).

### Example 5: Comparison of gene transduction efficiency according to use of enhancer

Gene transduction efficiency in D7F-TD10 conditions was evaluated using enhancers that increase lentiviral transduction efficiency, and the detailed experimental conditions are shown in Table 11.

**[Table 11] Time point of freeze, transduce, and check expression of NK cells in culture**

| **NK cells** | **Freezing** | **Transduction** | **Enhancers** | **Confirmation of CAR expression (+6 days after transduction)** |
|---|---|---|---|---|
| **TD3** | Not applicable | Day 3 | (5Z)-7-Oxozeaenol or BX795 | Day 9 |
| **TD10** | Not applicable | Day 10 | | Day 16 |
| **D7F-TD10** | Day 7 | Day 10 (Thaw + 3 days) | | Day 16 |

### 5-1) Lentiviral vector transduction using enhancers and FACS expression analysis

Umbilical cord blood-derived NKs were cultured using feeder cells in the same manner as in Example 1-1 and transfected with lentiviral vectors expressing HER2 CAR and IL-15 genes simultaneously at day 3, day 10, and D7F-TD10 conditions, respectively. The detailed gene transduction method was the same as in Examples 1-2, where NK cells were treated with 20 MOI of lentiviral vectors expressing HER2 CAR and IL-15, 10 µg/mL Protransduzin, and 20 ng/mL human IL-21 recombinant protein. In addition, NK cells were treated with (5Z)-7-oxozeaenol at a concentration of 6 µM or the TBK1 inhibitor BX795 (TOCRIS, 4318) at a concentration of 1.5 µM as an enhancer to increase lentiviral transduction efficiency. The day after transduction, fresh NK cell culture medium containing 20 ng/mL of IL-21 and 6 µM (5Z)-7-oxozeaenol or 1.5 µM BX795 was added in equal amounts to each well of the plate and incubated. NK cells cultured for 6 days after transduction were analyzed for HER2 CAR expression as a % value using the same method as in Examples 1-3.

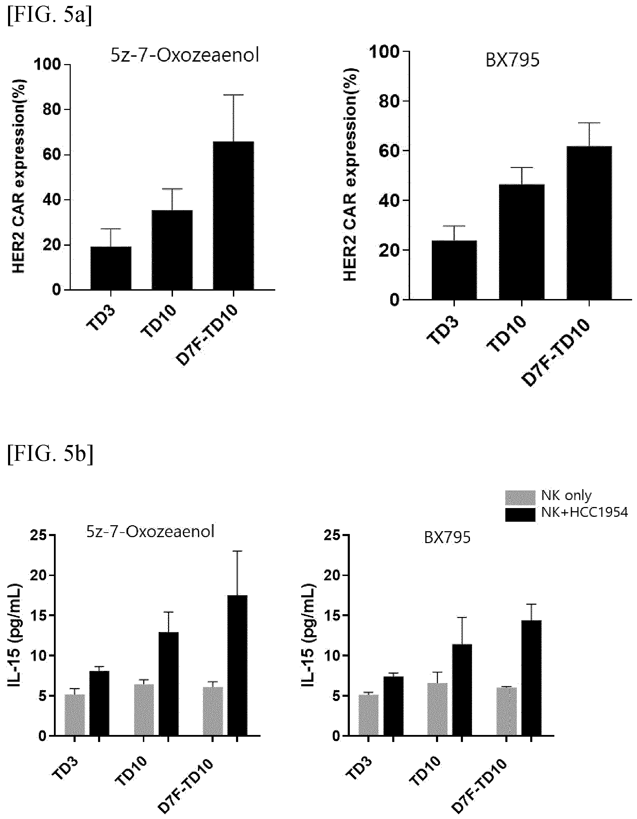

Regardless of the type of enhancer used, both enhancers resulted in the highest HER2 CAR expression in NKs frozen on day 7 and transfected on day 10 of culture (D7F-TD10) compared to TD3 and TD10 (FIG. 5a).

### 5-2) Cell culture to confirm IL-15 secretion in transduced cells

NK cells were cultured for 6 days after transduction, and HER2 CAR-positive cells were isolated for confirmation of IL-15 expression as follows. NK cells were centrifuged at 1,200 rpm for 5 minutes, the supernatant was removed and resuspended in MACS buffer (PBS with 0.5% FBS, 2 mM EDTA) to a cell concentration of 1 x 10⁷ /mL. Biotin-tagged recombinant human HER2 protein (Acrobiosystems, HE2-H822R) at a concentration of 50 ng/mL was added at 1.5 µL per 1 x 10⁶ of NK cells and incubated at 4°C for 30 min. MACS buffer equal to 10 times the volume of NK cells was added and centrifuged at 340g, 4°C, 10 minutes to remove the supernatant. After resuspending the cells in MACS buffer to a concentration of 1 x 10⁸ /mL, anti-biotin beads (Miltenyi Biotec, 130-092-357) were added at 37.5 µL per NK cell in 4 x 10⁷ /mL and incubated at 4°C for 30 min. MACS buffer equal to 10 times the volume of NK cells that reacted with the beads was added and centrifuged at 340g, 4°C, 10 minutes to remove the supernatant.

LS columns (Miltenyi Biotec) were loaded onto a QuadroMACSTM Separator (Miltenyi Biotec) and 3 mL of MACS buffer was run to activate the columns. The NK cells that reacted with the beads were well released by pipetting and loaded 500 µL per column and washed three times by running 3 mL of MACS buffer through the column. After washing, the columns were removed from the QuadroMACSTM Separator, 5 mL of MACS buffer was added, and the CAR-positive cells in the columns were separated using a plunger. The separated cells were suspended in NK cell culture medium at a concentration of 1 x 10⁶ /mL after centrifugation and placed in appropriate size well plates and cultured for another 13 days after restimulation with feeder cells in the same manner as in Example 1-1. NK cells cultured for a total of 29 days were frozen and stored in an ultra-low temperature liquid nitrogen tank until use.

### 5-3) Confirmation of IL-15 secretion

To determine the expression of IL-15 introduced simultaneously with the HER2 CAR, frozen NK cells were thawed on day 29 of culture and the amount of IL-15 secreted after co-culture with the target cancer cell line was measured by ELISA. HCC1954 (American Type culture collection, Manassas, VA, USA), a HER2-positive human breast cancer cell, was used as the target cancer cell line. The target cells were centrifuged with assay medium (RPMI 1640 with 10% FBS, Gibco, 11875093) at 1,200 rpm for 5 min, the supernatant was removed, and the cell pellet obtained after washing was resuspended in assay medium at a concentration of 6 x 10⁵ /mL. Frozen NK cells were prepared at a concentration of 2 x 10⁶ /mL in assay medium after thawing. A 96-well round-bottom plate was prepared and 100 µL of the prepared target cells and NK cells were added to each well for co-culture. For the NK cells alone control, 100 µL of assay medium was added with 100 µL of NK cells instead of target cells. After 24 hours of co-culture, the plate was taken out, centrifuged at 2000 rpm, 4°C for 3 minutes, 170 µL of the supernatant was collected, and the expression of IL-15 was measured with an IL-15 ELISA kit (R&D systems, S1500). In summary, the highest value standard was prepared at 250 pg/mL and then diluted by 1/2, resulting in a total of seven standards (250, 125, 62.5, 31.25, 15.63, 7.81, 3.91 pg/mL), including the lowest value of 3.9 pg/mL. In a 96-well plate pre-coated with the antibody provided in the ELISA kit, 50 µL of sample and standard were added to each well, and 50 µL of dilution buffer was added to the standard blank well. After 3 hours at room temperature, each well was washed four times with 300 µL of wash buffer. Human IL-15 conjugate was added to the wells in 200 µL increments and left for 45 minutes at room temperature. Each well was washed four times with 300 µL of wash buffer. The substrate solution, made by mixing equal parts of Solution A and Solution B, was added 200 µL to the wells and left for 30 minutes at room temperature, and the stop solution was added 50 µL to the wells and mixed gently to mix the solutions. The absorbance was measured at 450 nM wavelength using a Spectra max 190 instrument to calculate the IL-15 expression in the samples.

When co-cultured with HER2 CAR-positive target cells, HCC1954, the D7F-TD10 condition, which was frozen and thawed on day 7 and gene introduced on day 10, showed the highest IL-15 expression. On the other hand, the control condition with NK cells alone without target cells showed low IL-15 expression below 5 pg/mL with no significant difference according to the time of gene introduction (FIG. 5b). From the above results, it is indicated that the D7F-TD10 method showed the highest gene transduction efficiency even when using various enhancers that increase the transduction efficiency of lentiviral vectors (FIG. 5).

### Example 6: Comparison of lentiviral vector transduction efficiency and vector copy number (VCN)

The gene transfer efficiency of the D7F-TD10 condition was evaluated according to the type of delivery system used for lentiviral transduction. The detailed experimental conditions are shown in Table 12.

**[Table 12] Time point of freeze, transduce, and check expression of NK cells in culture**

| **NK cells** | **Freezing** | **Transduction** | **Transduction reagents** | **Confirmation of CAR expression (+6 days after transduction)** |
|---|---|---|---|---|
| **TD3** | Not applicable | Day 3 | Protransduzin | Day 9 |
| **D7F-TD10** | Day 7 | Day 10 (Thaw + 3 days) | | Day 16 |
| **D7F-TD10(L)** | Day 7 | Day 10 (Thaw + 3 days) | Lentiboost-P | Day 16 |

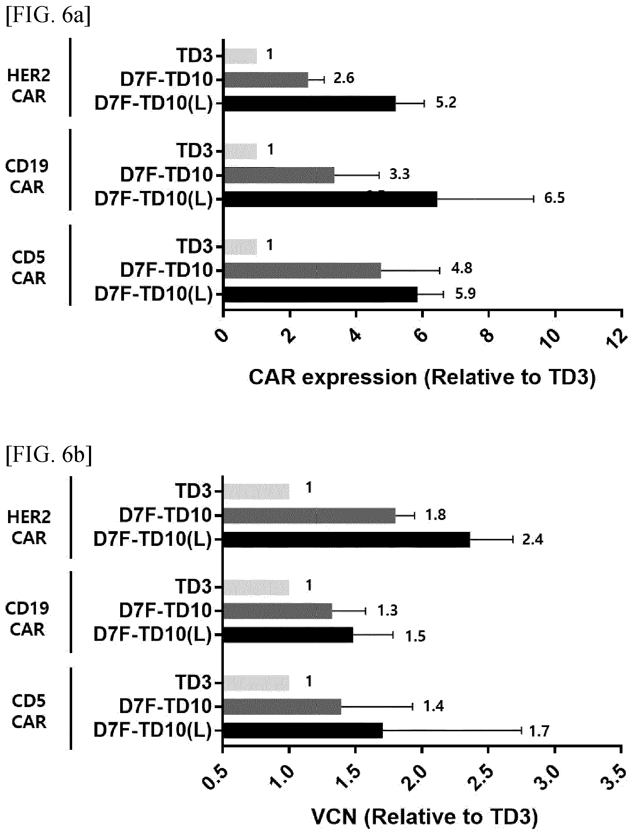

NK cells in TD3 and D7F-TD10 conditions were transfected with lentiviral vectors expressing HER2 CAR, CD5 CAR, and CD19 CAR in the same manner as in Example 3. For the D7F-TD10(L) condition, NK cells were suspended in NK cell culture medium to a concentration of 1 x 10⁶ /mL and transfected with lentiviral vectors at 20 MOI in the presence of Lentiboost-P (sirion biotec, P-grade) at 1/100 of the total volume and human IL-21 recombinant protein (Biolegned) at a concentration of 20 ng/mL. As a negative control, NK cells without lentiviral vector were used. Three lentiviral vectors expressing anti-HER2 scFv CAR (HER2 CAR), anti-CD5 scFv CAR (CD5 CAR), and anti-CD19 scFv CAR (CD19 CAR) genes were used, all produced by Lentigen (MD, USA). The expression of HER2 CAR, CD5 CAR, and CD19 CAR on NK cells cultured for 6 days after transduction was confirmed by the same method as in Examples 1-3. After the secondary staining, the NK cells were fixed with 200 µL fixation solution, and the CAR protein expression on the NK cell surface was analyzed by FACS equipment as a % value, with the TD3 value that showed the lowest expression being converted to 1. The expression values of D7F-TD10 or D7F-TD10(L) using Protransduzen or Lentiboost-P as the delivery reagent were calculated as a ratio to the TD3 cell expression, which was scaled to 1. The comparison of the gene expression revealed that all the various scFv CARs showed high gene expression efficiency under D7F-TD10 conditions regardless of the type of delivery reagent (FIG. 6a).

In addition, to measure VCN to confirm the degree of integration of the lentiviral vector in NK cells, genomic DNA was extracted from the same NK cells used in the above experiments and real-time PCR was performed. Genomic DNA extraction was performed using the NucleoSpin^{®} Tissue (MachereyNagel, 740952.250) kit, and the concentration of the final extracted genomic DNA was measured with a nano-drop instrument, followed by dilution of the DNA concentration to between 10-50 ng/uL using nuclease pre-water (Ambion, AM9937). The standard DNA, primers, and probe information used for real-time PCR is summarized in Table 13 and Table 14 below.

**[Table 13] Information of standard DNA for VCN Measurement**

| Target Gene | Plasmids / Insertion Genes | Modification |
|---|---|---|
| Anti-HER2 scFv CAR | pEF1a/ Anti-HER2 scFv CAR DNA | Cleaved and purified with NheIenzyme |
| Anti-CD19 or CD5 scFv CAR | pCR^{™} 4BluntTOPO/CAR signal domain | Cleaved and purified by PstI enzyme |
| RPPH1 | pT7 / RPPH1 | Cleaved and purified by PstI enzyme |

**[Table 14] Real-time PCR primers and probes**

| Anti-HER2 scFv CAR-transduced NK cells | | |
|---|---|---|
| Target Gene | Primers, Probes | Sequence (5'→3') |
| Anti-HER2 scFv CAR | Forward | ggagttcaaggggcagattcg |
| | Reverse | cccagtagtccactctcacg |
| | Probe | fam-aaggccgaggacaccgccgccgt-bhq1 |
| RPPH1 | Forward | ccctagtctcagaccttcccaag |
| | Reverse | gggggaggggaagctcatcag |
| | Probe | hex-ccacgagctgagtgcgtcctgtca-bhq 1 |

| Anti-CD19 or CD5 scFv CAR-transduced NK cells | | |
|---|---|---|
| Target Gene | Primers, Probes | Sequence (5'→3') |
| Anti-CD19 or CD5 scFv CAR | Forward | cgggtccccactcgcaaacac |
| | Reverse | cgccctacaggtcagagaga |
| | Probe | fam-ggcagcgttgccgacattct-zen |
| RPPH1 | Forward | ccctagtctcagaccttcccaag |
| | Reverse | gggggaggggaagctcatcag |
| | Probe | fam-ccacgagctgagtgcggtcctgtca-zen |

To measure the VCN of anti-HER2 scFv CAR, standard DNA stocks of anti-HER2 scFv CAR and RPPH1 (4 x 10⁷ copies/uL) were mixed equally and then diluted stepwise with nuclease pre-water to prepare 2 x 10 (STD1), 2 x 102 (STD2), 2 x 103 (STD3), 2 x 10⁴ (STD4), and 2 x 10⁵ (STD5) copies/µL. Spiking controls were prepared by mixing a 1/5 dilution of STD3 with an equal amount of genomic DNA (25 ng/µL) from wild-type NK cells. PCR controls for anti-HER2 scFv CAR targets were prepared with 10 ng/µL of genomic DNA from wild-type NK cells, and PCR blank controls used nuclease pre-water. Genomic DNA of the NK cells for which VCN is to be measured was prepared at 10 ng/µL diluted in nuclease premix. PCR premixes were prepared at the volumes indicated in Table 15 for each well for the target gene.

**[Table 15] PCR reagent for VCN measurement of Anti-HER2 scFv CARs**

| Reagents | | CAR Targets | RPPH1 Target |
|---|---|---|---|
| Premix volume per well | 2X Taqman Gene Expression Master Mix (applied biosystems, 4369510) | 12.5 µL | 12.5 µL |
| | Primer forward and reverse mix (10 µM) | 2.25 µL | 0.75 µL |
| | Probe (10 µM) | 0.32 µL | 0.63 µL |
| | Nuclease free water | 4.93 µL | 6.12 µL |
| Templated Volume per Well | | 5 µL | 5 µL |
| Total volume per well | | 25 µL | 25 µL |

MicroAmp^{®} Optical 96-well reaction plates (Applied biosystems, N8010560) were prepared, 5 µL of PCR template and 20 µL of premix were dispensed into each well, and PCR was performed with the cycles in Table 17. Based on the standard curve, the copy number of CAR and RPPH1 genes of each sample was calculated, and the number of cells was estimated by the calculated RPPH1 copy number, and the copy number of CAR was divided by the estimated number of cells to calculate the VCN of CAR gene per NK cell. The VCN was calibrated to the CAR expression rate (%) of each NK cell to calculate the final VCN.

The method for measuring VCN of anti-CD5 and CD19 scFv CARs was the same as the method for measuring anti-HER2 scFv VCN above: prepare a MicroAmp^{®} Optical 96-well reaction plate (Applied biosystems, N8010560) by preparing the standard plasmids in Table 13, primers and probes in Table 14, and PCR premixes in the volumes indicated in Table 16, dispense 5 µL of PCR template and 20 µL of premix into each well, and perform PCR with the cycles in Table 17.

**[Table 16] PCR reagent for VCN measurement of Anti-CD5 and Anti CD19 scFv CARs**

| Reagents | | CAR Targets | RPPH1 Target |
|---|---|---|---|
| Premix per well volume | 2X Taqman Gene Expression Master Mix (applied biosystems, 4369510) | 12.5 µL | 12.5 µL |
| | Primer and probe mix (10X) | 2.5 µL | 2.5 µL |
| | Nuclease free water | 5 µL | 5 µL |
| Template volume per well | | 5 µL | 5 µL |
| Total volume per well | | 25 µL | 25 µL |

**[Table 17] PCR cycle for VCN measurement**

| Stage | Temperature (°C) | Time | Cvcles |
|---|---|---|---|
| Hold | 50 | 2 minutes | 1 |
| | 95 | 10 minutes | 1 |
| PCR | 95 | 15 seconds | 40 |
| | 60 | 1 minute | |

VCN is a measure of gene transduction that counts the number of lentiviral vectors inserted into a single cell. The VCN of the transfected condition (TD3) on day 3 of culture was scaled to 1, and the relative VCN of each condition was calculated as a ratio to the TD3 VCN to compare the differences. All of the various scFv CARs showed higher VCN values when the gene was transduced by the D7F-TD10 method than TD3, regardless of the type of transduction (FIG. 6b). From the above results, it was concluded that NK cells frozen on day 7 of culture, thawed and cultured for 3 days, and transduced with lentiviral vectors on day 10 of culture, exhibits high gene introduction and expression efficiency.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method for expressing a target gene in an immune cell comprising:
(a) Freezing the immune cell;
(b) thawing the frozen immune cell; and
(c) introducing a gene delivery system comprising the target gene into the thawed immune cells.

2. The method of claim 1, wherein the method further comprises culturing the immune cell isolated from the subject *in vitro* for 6 to 11 days prior to the step (a).

3. The method of claim 1, wherein the method further comprises culturing the thawed immune cell for 2 to 4 days after the step (b).

4. The method of claim 1, wherein the immune cell is selected from the group consisting of T cell, natural killer (NK) cell and natural killer T (NKT) cell.

5. The method of claim 1, wherein the gene delivery system is a viral vector.

6. The method of claim 5, wherein the virus is selected from the group consisting of lentiviruses, adenoviruses, adeno-associated viruses (AAVs), retroviruses, herpes simplex viruses and vaccinia viruses.

7. The method of claim 6, wherein the virus is a lentivirus.

8. The method of claim 1, wherein the step (c) is performed by adding one or more compounds selected from the group consisting of a compound represented by Formula 1, a compound represented by Formula 2 and pharmaceutically acceptable salts thereof:

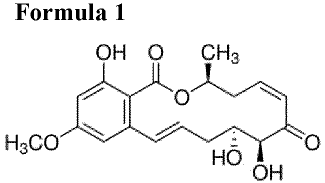

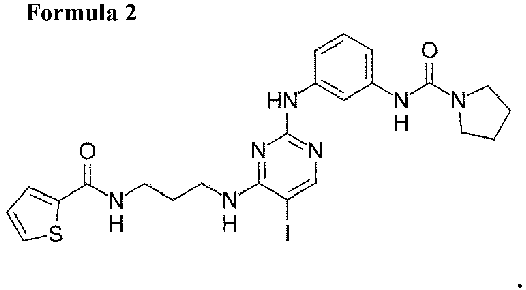

9. A natural killer cell expressing a target gene by the method of any one of claims 1 to 8.

10. A pharmaceutical composition for preventing or treating a tumor or infectious disease comprising the natural killer cell of claim 9 as an active ingredient.

11. A method for increasing the efficiency of a target gene expression in an immune cell, comprising freezing the immune cell; and thawing the frozen immune cell.

12. The method of claim 11, wherein the target gene is inserted into a viral vector and introduced into the immune cell.

13. The method of claim 12, wherein said virus is a lentivirus.

14. The method of claim 11, wherein the immune cell is selected from the group consisting of T cell, natural killer (NK) cell and natural killer T (NKT) cell.
